(19) Europäisches Patentamt

European Patent Office

Office européen des brevets

(11) Veröffentlichungsnummer : **0 067 409**
**B1**

(12) **EUROPÄISCHE PATENTSCHRIFT**

(45) Veröffentlichungstag der Patentschrift :
18.09.85

(51) Int. Cl.⁴ : **C 07 D307/60// C07C69/716**

(21) Anmeldenummer : **82105032.5**

(22) Anmeldetag : **08.06.82**

(54) Verfahren zur Herstellung von Tetronsäure.

(30) Priorität : **17.06.81 CH 3983/81**

(43) Veröffentlichungstag der Anmeldung :
**22.12.82 Patentblatt 82/51**

(45) Bekanntmachung des Hinweises auf die Patenterteilung : **18.09.85 Patentblatt 85/38**

(84) Benannte Vertragsstaaten :
**AT BE CH DE FR GB IT LI NL SE**

(56) Entgegenhaltungen :
**EP-A- 0 018 162**
**DE-A- 2 143 709**
**TETRAHEDRON, Band 34, Nr. 2, 1978, Seiten 223-231,
Oxford (GB) J.L. VAN DER BAAN et al.: "The total
synthesis of the antibiotic malonomicin (K16)".**

(73) Patentinhaber : **LONZA AG**

**Gampel/Wallis (CH)**

(72) Erfinder : **Miller, Raimund, Dr.**
**Berkshire Place 66**
**Hackensack, N.J. 07601 (US)**
Erfinder : **Tenud, Leander, Dr.**
**Balfrinstrasse 23**
**VISP (Kanton Wallis) (CH)**

(74) Vertreter : **Barz, Peter, Dr. et al**
**Patentanwälte Dr. V. Schmied-Kowarzik Dipl.-Ing. G.
Dannenberg Dr. P. Weinhold Dr. D. Gudel Dipl.-Ing. S.
Schubert Dr. P. Barz Siegfriedstrasse 8
D-8000 München 40 (DE)**

**Beschreibung**

Die Erfindung betrifft ein Verfahren zur Herstellung von Tetronsäure aus 4-Halogenacetessigester.

Es ist bekannt, Tetronsäure, die unter anderem als Beschleuniger für die photographische Entwicklung verwendet wird, aus 4-Monohalogenacetessigester oder -säure herzustellen. Nach CH Patent 503 722 wird 4-Chloracetessigester mit einem aromatischen Amin zum 3-Arylaminocrotolacton umgesetzt und aus diesem durch Behandlung mit Mineralsäuren die Tetronsäure in Freiheit gesetzt. Der Nachteil dieser Methode besteht darin, dass die Isolierung der Tetronsäure nur durch Hochvakuumsublimation zu realisieren ist.

Nach CH Patent 529 128 wird von 4-Halogenacetessigsäure ausgegangen, die in wässriger Lösung mit Alkalien umgesetzt wird. Durch Behandeln mit Mineralsäuren wird die Tetronsäure in Freiheit gesetzt. Auch hier muss die Isolierung der Tetronsäure über eine Hochvakuumsublimation erfolgen ; ausserdem ist die erzielbare Ausbeute nur 43-44 %.

Schließlich ist aus den Seiten 223 bis 231 von « Tetrahedron », 34, Nr. 2 (1978) bekannt, 4-Benzyloxyacetessigester aus 4-Bromacetessigester herzustellen.

Ziel der vorliegenden Erfindung ist es, diese Nachteile zu vermeiden.

Erfindungsgemäß wird dies dadurch erreicht, daß man aus dem entsprechenden 4-Benzyloxyacetessigester durch Hydrogenolyse den entsprechenden 4-Hydroxyacetessigester als Zwischenprodukt bildet, der durch Behandeln mit Säure in die Tetronsäure überführt wird.

Bei diesem erfindungsgemässen Verfahren kann der 4-Hydroxyacetessigester isoliert werden und nach Isolation die Behandlung mit Säure durchgeführt werden.

Es ist aber auch möglich, die Hydrogenolyse in Gegenwart von Säure durchzuführen. Dabei wird der primär in situ gebildete 4-Hydroxyacetessigester unmittelbar nach der Entstehung in die Tetronsäure überführt.

Unter Hydrogenolyse ist die Spaltung einer Verbindung durch Wasserstoff zu verstehen, wobei diese Wasserstoffbehandlung zweckmässig in Gegenwart von Hydrierungskatalysatoren und unter Druck stattfindet. Solche Hydrierungskatalysatoren sind beispielsweise Edelmetalle wie Platin, Ruthenium, Rhodium, Raneynickel, Kobalt und dergleichen, die zur Erhöhung der reagierenden Oberfläche zweckmässig auf Träger, z. B. Bimstein, Kohle, Silicagel, Tonerde und andere mehr, aufgebracht sind.

Die bevorzugten Temperaturen für die Hydrogenolyse sind 0 bis 30 °C.

Zweckmäßig wird ein Benzyloxyacetessigester eingesetzt, der aus einem 4-Halogenacetessigester hergestellt worden ist. Als 4-Halogenacetessigester kommen daher die 4-Brom- und 4-Chlorderivate, vorzugsweise die 4-Chloriderivate, zum Einsatz. Als Ester werden zweckmäßig solche von Alkoholen mit 1 bis 6 C-Atomen, wie methyl-, Äthyl-, Propyl-, Butylalkohol verwendet. Vorzugsweise wird 4-Chloracetessigäthylester verwendet.

Die Überführung des 4-Halogenacetessigesters in den 4-Benzyloxyacetessigester geschieht zweckmäßig durch Einwirkenlassen eines Metallsalzes, insbesondere eines Alkalisalzes, vorgzugsweise des natriumsalzes, des Benzylalkohols auf den 4-Halogenacetessigester in einem organischen Lösungsmittel. Als Lösungsmittel können alle geeigneten Verbindungen Verwendung finden, vorteilhaft aber werden Dimethylsulfoxid und Tetrahydrofuran verwendet. Die Reaktionstemperatur wird zweckmäßig bei -10 bis + 50 °C gehalten.

Eine bevorzugte Ausführungsform besteht darin, dass man von Natriumhydrid ausgeht, dieses in Tetrahydrofuran suspendiert und den Benzylalkohol zudosiert. In die so erhaltene Lösung wird der 4-Halogenacetessigester, gelöst in Tetrahydrofuran, zudosiert. Die dabei zweckmässigste Reationstemperatur liegt bei 0 bis 40 °C.

Nach beendeter Reaktion kann das Tetrahydrofuran durch Destillation zurückgewonnen werden. Der nach dieser Arbeitsweise hergestellte 4-Benzyloxyacetessigäthylester ist ein viskoses Oel mit einem $Kp_{0,5}$ von 135 bis 136 °C.

Die Säurebehandlung des sich als Zwischenprodukt bildenden 4-Hydroxyacetessigesters, während oder nach der Hydrogenolyse, geschieht zweckmässig bei Temperaturen von 0 bis 30 °C. Als Säure kommen Salzsäure, Trifluoressigsäure, saure Kationenaustauscher usw. in Frage. Vorzugsweise wird Salzsäure in halbkonzentrierter Form verwendet.

Beispiel 1

7,56 g 80 %iges Natriumhydrid wurden durch dreimaliges Waschen mit je 30 ml Petroläther (Kp 40 bis 60 °C) vom Weissöl befreit und zu 160 ml Tetrahydrofuran gegeben. Unter Rühren wurden nun 14,28 g Benzylalkohol so zudosiert, dass eine Raktionstemperatur von 40 °C eingehalten wurde. Nach beendeter Wasserstoffentwkicklung wurde whährend 1 Stunde eine Lösung von 19,74 g (95,5 %ig = 0,114 5 Mol) 4-Chloracetessigäthylester in 80 ml Tetrahydrofuran zugetropft, wobei eine Temperatur von 40 °C herrschte. Nach 15 stündigem Rühren, wobei die Temperatur Raumtemperatur annahm, wurde die Hälfte des Tetrahydrofurans am Vakuumrotationsverdampfer abgedampft und der noch fliessbare Rückstand in dünnem Strahl in eine Mischung von 14,3 g konz. HCl in 150 g Eiswasser gegossen, wobei sich der pH nach Ende der Zugabe auf 5 einstellte. Nun wurde 4 mal mit Aether extrahiert und nach Waschen und Trocknen der Aether am Vakuumrotationsverdampfer abgedampft. Der Rückstand wurde destilliert. Es resultierten 22,52 g (83,2 %) 4-Benzyloxyacetessigäthylester (Kp$_{0,4}$ 126 °C).

21,24 g dieses Esters wurden, gelöst in essigester (100 ml) in einem 200 ml-Stahlautoklaven, mit Magnetrührer versehen, eingebracht und in Gegenwart von 1,05 g Pd 5 % auf Kohle mit Wasserstoff bei einem Druck von 5 atm hydrogenolysiert. Nach beendeter Reaktion, die etwa 2 Stunden betrug, und nach einer 2-Stündigen Nachreaktionszeit wurde der Katalysator abfiltriert und das Filtrat am Vakuumrotationsverdampfer bei 35 °C eingedampft. Der Rückstand wurde im Hochvakuum getrocknet. Es resultierten 13,4 g 4-Hydroxyacetessigäthylester in Form eines leicht gelblichen Oeles.

4,43 g dieses Esters wurden in 10 ml 18 %iger HCl gelöst und während 6 Stunden bei Raumtemperatur gerührt. Anschliessend wurde die HCl am Vakuumrotationsverdampfer bei Temperatur unter 30 °C abgezogen, der kristalline Rückstand in wenig Wasser gelöst und das Wasser am Vakuumrotationsverdampfer bei Temperatur unter 30 °C wieder abgezogen. Diese Operation wurde noch einmal wiederholt. Es resultierten 3,0 g Tetronsäure in einer Reinheit von 95,8 %, das entsprach einer Ausbeute von 95,8 %, bezogen auf den 4 Benzyloxyester.

Die Gesamtausbeute, bezogen auf den 4-Chlorester, betrug 79,7 %.

## Beispiel 2

Es wurde 4-Benzyloxyacetessigäthylester wie in Beispiel 1 hergestellt. Die Hydrogenolyse mit Pd auf Kohle wurde jedoch in Gegenwart von Aethanol als Lösungsmittel und in Gegenwart von konz. HCl durchgeführt. Es resultierte dabei direkt Tetronsäure in einer Ausbeute, die nur wenig unter der des Beispiel 1 lag.

## Beispiel 3

Wie in Beispiel 1 wurde das Natriumbenzylat in Tetrahydrofuran mit 4-Chloracetessigsäure-isopropylester zur Reaktion gebracht. Der resultierende 4-Benzyloxyacetessigsäure-isopropylester wurde anschliessend hydrogenolysiert. Der so gebildete 4-Hydroxyacetessigsäure-isopropylester wurde durch Behandlung mit 18 %ger HCl bei Raumtemperatur in Tetronsäure überführt. Gesamtausbeute, bezogen auf 4-Chloracetessigsäure-isopropylester : 78 %.

## Beispiel 4

Wie in Beispiel 1 wurde das Natriumbenzylat in Tetrahydrofuran mit 4-Chloracetessigsäure-n-butylester zur Reaktion gebracht. Der resultierende 4-Benzyloxyacetessigsäure-n-butylester wurde anschliessend hydrogenolysiert. Der so gebildete 4-Hydroxyacetessigsäure-n-butylester wurde durch Behandlung mit 18 %iger HCl bei Raumtemperatur in Tetronsäure überführt.

Gesamtausbeute, bezogen auf 4-Chloracetessigsäure-n-butylester : 76,5 %.

## Patentansprüche

1. Verfahren zur Herstellung von Tetronsäure aus 4-Halogenacetessigester, dadurch gekennzeichnet, daß man aus dem entsprechenden 4-Benzyloxyacetessigester durch Hydrogenolyse den entsprechenden 4-Hydroxyacetessigester als Zwischenprodukt bildet, der durch Behandeln mit Säure in die Tetronsäure überführt wird.

2. Verfahren nach Patentanspruch 1, dadurch gekennzeichnet, daß man den 4-Hydroxyacetessigester isoliert.

3. Verfahren nach Patentanspruch 1, dadurch gekennzeichnet, daß man die Hydrogenolyse in Gegenwart von Säure durchführt, wobei der sich in situ bildende 4-Hycroxyacetessigester direkt in die Tetronsäure umgelagert wird.

4. Verfahren nach Patentanspruch 1, dadurch gekennzeichnet, daß man einen 4-Benzyloxyacetessigester einsetzt, der aus einem 4-Halogenacetessigester hergestellt worden ist.

5. Verfahren nach den Patentansprüchen 1, 2 und 3, dadurch gekennzeichnet, daß man die Hydrogenolyse unter Druck und in Gegenwart von Hydrierungskatalysatoren durchführt.

## Claims

1. Process for the production of tetronic acid from 4-haloacetoacetic acid ester, characterized in that the corresponding 4-hydroxyacetoacetic acid ester is formed as intermediate from the respective 4-benzyloxyacetoacetic acid ester by hydrogenolysis which hydroxy-acetoacetic acid ester is converted into tetronic acid by treatment with acid.

2. Process according to patent claim 1, characterized in that the 4-hydroxyacetoacetic acid ester is isolated.

3. Process according to patent claim 1, characterized in that the hydrogenolysis is carried out in the presence of acid, the 4-hydroxyacetoacetic acid ester, which is formed in situ, being directly rearranged into the tetronic acid.

4. Process according to patent claim 1, characterized in that a 4-benzyloxyacetoacetic acid ester is used which has been prepared from a 4-haloacetoacetic acid ester.

5. Process according to patent claims 1, 2 and 3, characterized in that the hydrogenolysis is carried out under pressure and in the presence of hydrogenation catalysts.

## Revendications

1. Procédé pour la préparation de l'acide tétronique à partir d'esters d'acides 4-halogénoacétylacétiques, caractérisé en ce qu'on forme, à partir de l'ester d'acide 4-benzyloxyacétylacétique correspondant, par hydrogénolyse, l'ester d'acide 4-hydroxyacétylacétique correspondant en tant que produit intermédiaire qui est transformé en l'acide tétronique par traitement à l'acide.

2. Procédé selon la revendication 1, caractérisé en ce que l'on isole l'ester d'acide 4-hydroxyacétylacétique.

3. Procédé selon la revendication 1, caractérisé en ce que l'on effectue l'hydrogénolyse en présence d'acide, l'ester d'acide 4-hydroxyacéty-lacétique se formant in situ étant directement transposé en l'acide tétronique.

4. Procédé selon la revendication 1, caractérisé en ce que l'on met en œuvre un ester d'acide 4-benzyloxyacétylacétique qui a été préparé à partir d'un ester d'acide 4-halogénoacétylacétique.

5. Procédé selon les revendications 1, 2 et 3, caractérisé en ce que l'on effectue l'hydrogénolyse sous pression et en présence de catalyseurs d'hydrogénation.